# EUROPEAN PATENT APPLICATION

(11) **EP 3 989 236 A1**
(43) Date of publication of application: **27.04.2022**
(21) Application number: 20203489.8
(22) Date of filing: 23.10.2020
(51) Int. Cl.: G16H 40/60

(54) **SYSTEM FOR A MICROSCOPE SYSTEM AND CORRESPONDING METHOD AND COMPUTER PROGRAM**

(71) Applicant: Leica Instruments (Singapore) Pte. Ltd., 608924 Singapore (SG)
(72) Inventor: KOK, Alvin, Singapore 085301 (SG); PAN, Jiahao, Singapore 764504 (SG)
(74) Representative: 2SPL Patentanwälte PartG mbB

(57) **Abstract**

Examples relate to a system for a microscope system, to a microscope system comprising such a system, and to a corresponding method and computer program. The system comprises one or more processors and one or more storage devices. The system is configured to obtain a trigger signal. The trigger signal indicates a desire of a user of the microscope system to display a visual representation of a control functionality associated with an input device of the microscope system. The system is configured to generate, based on the trigger signal, a visual overlay comprising the visual representation. The system is configured to provide a display signal to a display device of the microscope system, the display signal comprising the visual overlay.

## Description

### Technical field

Examples relate to a system for a microscope system, to a microscope system comprising such a system, and to a corresponding method and computer program.

### Background

Modern microscope systems, in particular surgical microscope systems, offer a wide variety of functionality to assist the user (i.e. surgeon) during operation of the microscope. At the same time, the user might prefer to keep their eyes at the eyepiece. This may complicate the operation of the microscope system, as the input devices used to control the various functionalities may be occluded from the user. Also, due to the large number of different functionalities available to the user, a user may be unsure about which input modality of an input device addresses which functionality.

### Summary

There may be a desire for providing an improved concept for a microscope system, in which the functionality is made more easily accessible to the user of the microscope system.

This desire is addressed by the subject-matter of the independent claims.

Embodiments of the present disclosure provide a microscope system and a corresponding system, method and computer program for a microscope system. Embodiments of the present disclosure are based on the finding, that during the operation of microscopes, and in particular of surgical microscopes, a large number of functionalities are at the disposal of the user of the microscope. For example, in surgical microscopes for use in ophthalmic surgery, each input device may comprise different input modalities (e.g. buttons, foot-actuated buttons, control sticks etc.), with different functionalities being assigned to the input modalities based on a mode of operation of the surgical microscope. Also, each user (e.g. surgeon) may be free to assign their preferred sets of functionalities to the input devices, such that there is no fixed relationship between an input modality and a (control) functionality of the microscope. Embodiments of the present disclosure provide a system for a microscope system, and a corresponding microscope system, method and computer program, that is configured to generate a visual overlay of the control functionality that is available to the user via a given input device. The visual overlay is provided to a display device of the microscope system, e.g. ocular displays or an auxiliary display, and can be perused by the user of the microscope to make sure of the control functionality being assigned to the various input modalities. To summon the visual overlay, the user can trigger the system using a trigger signal, which may be generated using the very input device that the control functionality is being displayed of, or which may be generated using a different system, such as voice control or a touch-based interface.

Various embodiments of the present disclosure relate to a system for a microscope system. The system comprises one or more processors and one or more storage devices. The system is configured to obtain a trigger signal. The trigger signal indicates a desire of a user of the microscope system to display a visual representation of a control functionality associated with an input device of the microscope system. The system is configured to generate, based on the trigger signal, a visual overlay comprising the visual representation. The system is configured to provide a display signal to a display device of the microscope system. The display signal comprises the visual overlay. The visual overlay comprising the visual representation of the control functionality associated with the input device of the microscope system may be used by the users of the microscope system, e.g. the surgeons, to keep informed about the control functionality being assigned to the various input modalities.

In some examples, the system is configured to obtain the trigger signal from one of one or more input devices of the microscope system, and to generate the visual overlay with a visual representation of the control functionality associated with the input device the trigger signal is received from. In other words, the visual overlay that is triggered may relate to the same input device that is used to generate the trigger. For example, the system may be configured to obtain the trigger signal from a button of the input device. This button may remain constant regardless of a mode of operation the microscope system is being used in.

Beside the input device itself, other modalities may be used to trigger the display of the overlay. For example, the system may be configured to process audio captured via a microphone of the microscope system, and to generate the trigger signal based on one or more keywords spoken by the user within the captured audio. In this implementation, the user/surgeon might not be required to remove a hand or foot from another input modality they are planning to use. Alternatively or additionally, the system may be configured to obtain the trigger signal via a touch-screen of the microscope system. For example, an assistant may trigger the visual aide via the touch screen.

Microscope systems, in particular surgical microscope systems, feature a wide range of different input devices. Accordingly, the visual representation may represent a control functionality associated with a foot pedal, with a handle of the microscope system, with a mouth switch of the microscope system, or with an eye-tracking system of the microscope system.

As has been pointed out above, microscope systems, and in particular surgical microscope systems, may be operated in different modes of operation, with the control functionality being assigned to the input modalities of the input devices varying between modes of operation. Accordingly, the input device may have (or may be configured to affect) two or more sets of functionalities that are associated with two or more modes of the input device, with one of the two or more modes being active at the input device. The system may be configured to generate the visual representation based on the set of functionalities that is associated with the mode that is active at the input device. In other words, the visual overlay may be adapted based on the mode of operation currently being used.

In some examples, the visual representation of the control functionality comprises a visual representation of the input device the control functionality is associated with. This may allow for a more comprehensive display of the associated control functionality, in particular with input devices having a complex shape, such as handles with input modalities at different sides.

As mentioned before, the control functionality being available through a microscope system, and in particular through a surgical microscope system, is constantly expanding. However, at the same time, only a limited number of different input modalities of the input device are available. To support a greater number of functionalities, an "unlimited selection of functionalities" may be used, via a further trigger signal. In other words, the system may be configured to obtain a further trigger signal, the further trigger signal indicating a desire of a user of the microscope system to toggle a control functionality associated with the input device. The system may be configured to toggle the control functionality associated with the input device based on the further trigger signal. The system may be configured to adapt the visual representation after toggling the control functionality associated with the input device. By toggling the control functionality, a greater number of functionalities may be available via the input device, while the visual overlay may be used to indicate the input modalities of the input device being associated with the newly selected (i.e. toggled) functionality.

The control functionality being available for selection, and the effect of the selection, may be highlighted via the overlay. In other words, the system may be configured to generate the visual overlay such, that the visual overlay comprises a visual representation of a control functionality currently associated with the input device, and a visual representation of a control functionality available for association with the input device after toggling.

As has been pointed out before, the input device may comprise a plurality of input modalities, such as at least one of one or more buttons, one or more pads, one or more control sticks, one or more rotary controls etc. Accordingly, the visual representation may represent a plurality of control functionalities associated with the plurality of input modalities of the input device.

Various embodiments of the present disclosure further provide the microscope system comprising the system, an input device, and a display device. For example, the input device may be one of a foot pedal, a handle, a mouth switch or an eye-tracking system of the microscope system. Similar to above, the input device may comprise a plurality of input modalities, the plurality of input modalities comprising at least one of one or more buttons, one or more switches, one or more rotary controls, and one or more control sticks.

Various embodiments of the present disclosure further provide a corresponding method for a microscope system. The method comprises obtaining a trigger signal. The trigger signal indicates a desire of a user of the microscope system to display a visual representation of a control functionality associated with an input device of the microscope system. The method comprises generating, based on the trigger signal, a visual overlay comprising the visual representation. The method comprises providing a display signal to a display device of the microscope system, the display signal comprising the visual overlay.

Various embodiments of the present disclosure further provide a corresponding computer program with a program code for performing the method when the computer program is executed on a processor.

### Short description of the Figures

Some examples of apparatuses and/or methods will be described in the following by way of example only, and with reference to the accompanying figures, in which
- Fig. 1a: shows a block diagram of an example of a system for a microscope system;
- Fig. 1b: shows a schematic diagram of a surgical microscope system for use in ophthalmology;
- Fig. 2: shows a flow chart of an example of a method for a microscope system;
- Figs. 3a and 3b: show schematic diagrams of functionalities being assigned to a foot pedal of a surgical microscope system;
- Figs. 4a to 4c: show schematic diagrams of input devices for a microscope system, and of the input modalities of the input devices;
- Figs. 5a and 5b: show a schematic diagram of a surgical guidance overlay being displayed on a display device of a microscope system;
- Fig. 6: shows an example of an on-screen menu; and
- Fig. 7: shows an example of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

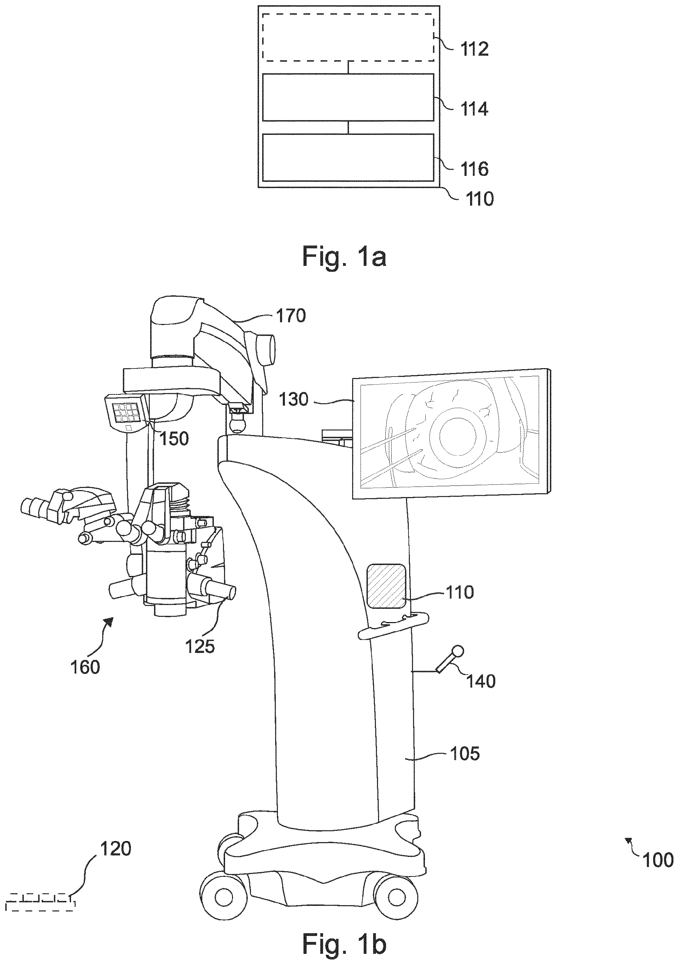

Fig. 1a shows a block diagram of an example of a system 110 for a microscope system 100. The system 110 comprises one or more processors 114 and one or more storage devices 116. Optionally, the system further comprises an interface 112. The one or more processors 114 are coupled to the optional interface 112 and the one or more storage devices 116. In general, the functionality of the system 110 is provided by the one or more processors 114, e.g. in conjunction with the optional interface 112 and/or the one or more storage devices 116.

The system is configured to obtain a trigger signal (e.g. via the interface 112). The trigger signal indicates a desire of a user of the microscope system to display a visual representation of a control functionality associated with an input device 120; 125 of the microscope system. The system is configured to generate, based on the trigger signal, a visual overlay comprising the visual representation. The system is configured to provide a display signal to a display device 130 of the microscope system (e.g. via the interface 112). The display signal comprises the visual overlay.

Various embodiments of the present disclosure further provide the microscope system 100, e.g. a surgical microscope system 100, comprising the system 110. Fig. 1b shows a schematic diagram of a surgical microscope system for use in ophthalmology comprising the system 100. The microscope system 100 further comprises one or more input devices 120; 125, e.g. a food pedal 120 and/or handles 125, the display device 130, and a microscope 160. The microscope system 110 may comprise one or more additional, optional features, such as a base unit 105 (that comprises the system 110), an arm 170 that the microscope 160 is attached to, or a microphone 140. For example, the microscope 160 may comprise ocular eyepieces 130. The microscope system shown in Fig. 1b is a surgical microscope system, which may be used at a surgical site by a surgeon. In particular, the microscope system shown in Fig. 1b is a surgical microscope system for use in ophthalmology (eye surgery), the same concept may, however, also be used in other types of surgical microscope systems, such as surgical microscope systems for use in neurosurgery. For example, the microscope system may comprise, or be used in combination, with one or more additional systems, such as an optical coherence tomography (OCT) system (not shown).

Embodiments of the present disclosure relate to a system, a method and a computer program that are suitable for a microscope system, such as the microscope system 100 introduced in connection with Fig. 1b. As has been introduced above, a distinction is made between the microscope 160 and the microscope system 100, with the microscope system comprising the microscope 160 and various components that are used in conjunction with the microscope 160, e.g. a lighting system, an auxiliary display etc. In a microscope system, the actual microscope is often also referred to as the "optical carrier", as it comprises the optical components of the microscope system. In general, a microscope is an optical instrument that is suitable for examining objects that are too small to be examined by the human eye (alone). For example, a microscope may provide an optical magnification of an object. In modern microscopes, the optical magnification is often provided for a camera or an imaging sensor. The microscope 160 may further comprise one or more optical magnification components that are used to magnify a view on the sample.

There are a variety of different types of microscopes. If the microscope system is used in the medical or biological fields, the object being viewed through the microscope may be a sample of organic tissue, e.g. arranged within a petri dish or present in a part of a body of a patient. For example, the microscope system 100 may be a microscope system for use in a laboratory, e.g. a microscope that may be used to examine the sample of organic tissue in a petri dish. Alternatively, the microscope 160 may be part of a surgical microscope system 100, e.g. a microscope to be used during a surgical procedure. Such a system is shown in Fig. 1b, for example. Although embodiments are described in connection with a microscope system, they may also be applied, in a more general manner, to any optical device. For example, the microscope system may be a system for performing material testing or integrity testing of materials, e.g. of metals or composite materials.

The system is configured to obtain the trigger signal, which indicates the desire of the user of the microscope system to display a visual representation of the control functionality associated with the input device 120; 125 of the microscope system. In general, the trigger signal may originate from two types of sources - either from the input device the visual representation is to be generated for, or from another input device or modality.

In the first case, the trigger signal may also indicate the input device that the visual representation is to be generated for. For example, the microscope system may comprise one or more input devices (e.g. a plurality of input devices). Depending on the input device the trigger signal originates from (i.e. the input device the trigger signal is obtained from), one of the one or more input devices may be selected, and the visual representation may be generated for the selected input device. In other words, the system may be configured to obtain the trigger signal from one of the one or more input devices 120; 125 of the microscope system, and to generate the visual overlay with a visual representation of the control functionality associated with the input device the trigger signal is received from (i.e. the input device that the trigger signal originates from). For example, if the trigger signal is received from a foot pedal of the microscope system, the visual representation may relate to said foot pedal. For example, the system may be configured to obtain the trigger signal from, or via, a button of the input device. In other words, the trigger signal may be generated in response to the button of the input device being actuated.

In the second case, the trigger device is received from, originates from, and is generated by, an input modality that is different from the input modality the visual representation is generated for. For example, the trigger signal may be generated by the system itself, by processing an input signal that is generated by a sensor of the microscope system. For example, the microscope system may comprise, or may be coupled with, a microphone 140. The system may be configured to process audio captured via the microphone 140, and to generate the trigger signal based on one or more keywords spoken by the user within the captured audio. For example, the system may be configured to detect the one or more keywords (or key phrases) within the captured audio, such as "show the functionality of the foot pedal", or "show an overview of the main input devices", or "how do I focus/zoom the microscope". The microscope system may comprise other types of sensors as well, such as a camera for performing eye tracking of the user, or a depth sensor for detecting gestures of the user. The system may be configured to process the sensor data of the camera or of the depth sensor, and to generate the trigger signal based on the sensor data. In some examples, the trigger signal may be generated via a touch-screen 150 of the microscope system. In other words, the system may be configured to obtain the trigger signal via a touch-screen 150 of the microscope system. For example, the touch-screen may be actuated by the user/surgeon themselves, or by an assistant, to generate the trigger signal.

In general, the proposed concept can be used with any type of input device. In (surgical) microscope systems, the two input devices that are predominately used are the foot pedal 120, and the handle or handles 125 of the microscope system. However, the same concept may be applied to other input devices as well, such as a mouth switch or an eye-tracking system of the microscope system. In other words, the input device may be one of a foot pedal 120, a handle 125, a mouth switch or an eye-tracking system of the microscope system. Accordingly, the visual representation may represent a control functionality associated with a foot pedal 120, with a handle 125 of the microscope system, with a mouth switch of the microscope system, or with an eye-tracking system of the microscope system. In any case, the input device, or the one or more input devices, may be an input device/one or more input devices comprising one or more input modalities that are re-configurable regarding the control functionality associated with the one or more input modalities. At the same time, the control functionality of the input device might not be shown on the input device itself. In particular, the input device might not be a touchscreen, as in touchscreens, the control functionality of an element of a graphical user interface might always be displayed on the screen.

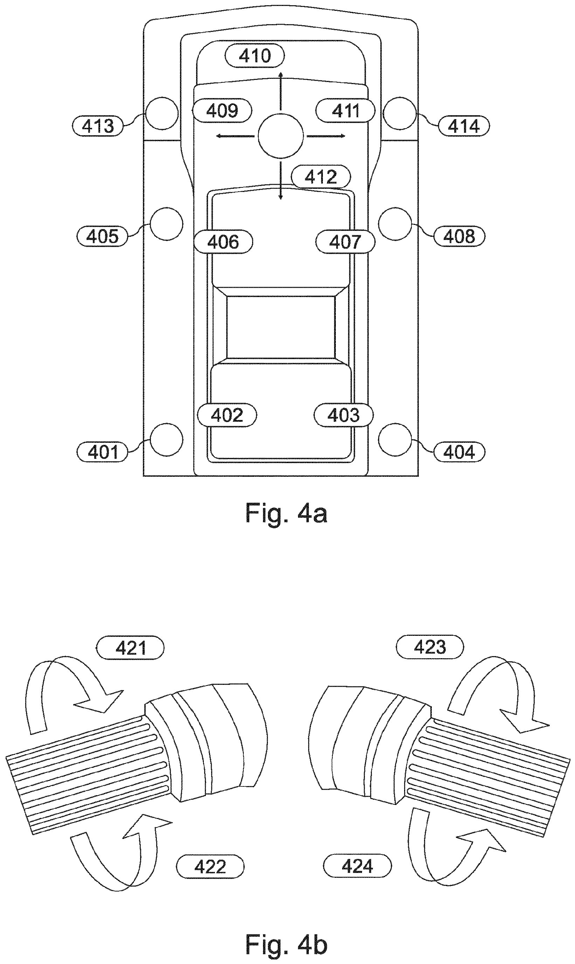

In the present application, a distinction is made between "input devices" and "input modalities". In general, an "input device" may relate to the device itself, such as a foot pedal, or a handle, and the "input modality" may relate to the means provided by the input device to trigger a control functionality. For example, the input device may comprise a plurality of input modalities, with the plurality of input modalities comprising at least one of one or more buttons, one or more switches, one or more rotary controls, and one or more control sticks. For example, the input device "foot pedal" 120 may comprise a plurality of input modalities, e.g. buttons that can be pressed, pads that can be tilted in one or another direction, or control sticks that can be tilted in one of multiple directions. For example, in Fig. 4a, a foot pedal is shown (i.e. the input device), with six buttons 401, 404, 405, 408, 413 and 414, two pads each being pressable to the left (402, 406) and to the right (403, 407), and a control stick that can be tilted left 409, upwards 410, right 411 and downwards 412, with the buttons, the directions the pads can be pressed and the directions the control stick can be tilted providing a total of 14 input modalities (6 via the buttons, 4 via the pads, and 4 via the control stick). If the input device is an eye-tracking system, the different directions of the gaze of the user may correspond to the input modalities. If the input device is a mouth switch, the input modalities may be an "actuation modality" (e.g. a switch to be actuated via the chin), and a four-way joystick to be actuated via the mouth.

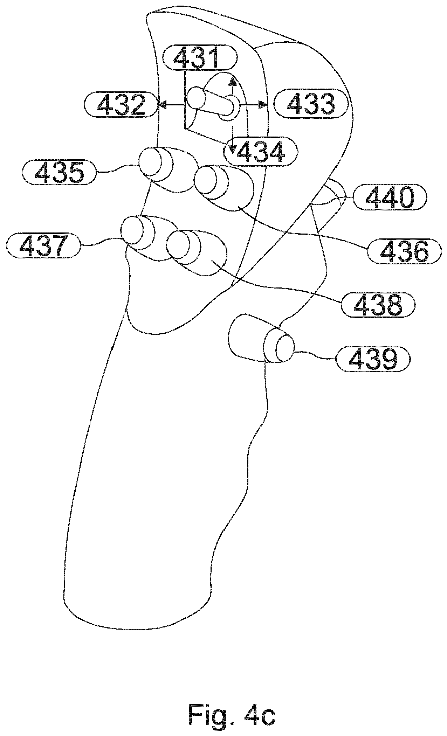

As is evident, the term "foot pedal" is not used for a single button that can be actuated by foot, but for the entire input device comprising the plurality of input modalities. Similarly, the handles shown in Fig. 4a have two input modalities each, a clockwise turn and a counter-clockwise turn. The handle of Fig. 4c has 10 input modalities - six buttons 435-440, and a control stick that can be turned into four directions (431-434). As is evident, a plurality of control functionalities can be associated with a plurality of input modalities of the input device. Accordingly, the visual representation may represent a plurality of control functionalities associated with a plurality of input modalities of the input device.

As is evident from the above examples, a large number of input modalities may be supported, with input devices that may have complex shapes, such as the control handle shown in Fig. 4c. To aid the comprehensibility of the visual overlay, a visual representation of the input device may be included in the visual representation, e.g. a three-dimensional visual representation. In other words, the visual representation of the control functionality may comprise a visual representation, such as a three-dimensional representation, of the input device the control functionality is associated with. In some examples, the display device 130 may be a display device for displaying three-dimensional images. For example, the display device may be configured to provide the three-dimensional images using different polarizations, with the user/surgeon wearing polarized glasses.

The trigger signal indicates the desire of a user of the microscope system to display the visual representation of the control functionality associated with the input device 120; 125 of the microscope system. In other words, the trigger signal is triggered, by the user, in order to display the display the visual representation of the control functionality associated with the input device. Accordingly, the visual representation is displayed in response to the trigger signal. In some examples, the visual representation may be shown (only) as long as the trigger signal is active. Alternatively, the visual representation may be shown for a pre-defined time in response to the trigger signal, and hidden after the pre-defined time. For example, the system may be configured to hide and/or fade out the visual representation after the pre-defined time.

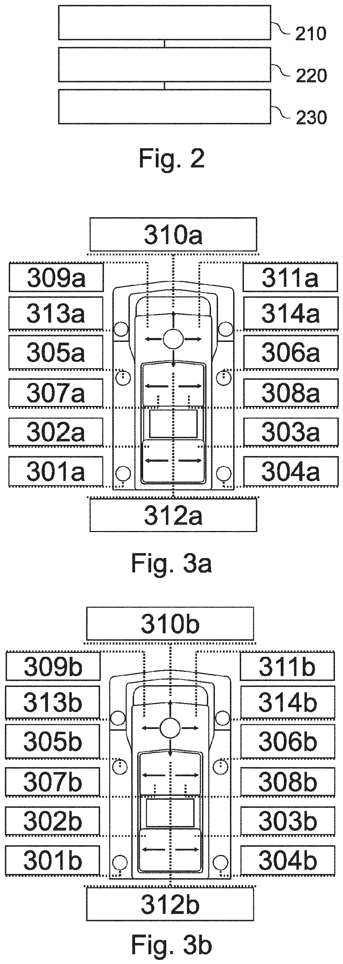

The system is configured to generate, based on the trigger signal, the visual overlay comprising the visual representation. In general, any kind of visual representation of the control functionality may be part of the visual overlay. For example, the visual representation may comprise a pictogram representation of the control functionality, or the visual representation may comprise a textual representation of the control functionality. In other words, for each control functionality accessible via an input modality of the input modality, the visual representation may comprise a textual or pictogram representation, e.g. as shown in Figs. 3a and 3b. For example, the visual overlay may comprise the visual representation of the control functionality associated with the input modalities in a manner that mimics the layout of the input device, e.g. with the visual representation of the control functionality being located in proximity of a visual representation of the input device itself, e.g. as shown in Figs. 3a to 4c. For examples, the visual representation may represent a control functionality associated with a foot pedal 120, with a handle 125 of the microscope system, with a mouth switch of the microscope system, or with an eye-tracking system of the microscope system, and/or, in particular, of with the input modalities provided by the respective input device.

In general, the control functionality may relate to any functionality of the microscope system that can be triggered or adapted, e.g. a zoom functionality, a focus functionality, a movement of a robotic arm of the microscope system, an image processing functionality of the microscope system, a display functionality of the microscope system, an illumination functionality of the microscope system (e.g. fluorescence stimulation or visible light illumination) etc. In some cases, the control functionality may also relate to further devices that are coupled with the microscope system, such as optical coherence tomography and intraocular lens guidance in the case of a surgical microscope system for use in eye surgery. In other words, the control functionality may relate to functionality provided by the microscope system and/or to functionality provided by a device that is coupled with, or integrated with, the microscope system, such as an OCT system or a lens guidance system.

As has been mentioned above, a microscope system, and in particular a surgical microscope system, may comprise more than a single mode of operation. For examples, a surgical microscope system for use in eye surgery may have a vitreoretinal mode and an OCT mode. In the different modes of operation, different control functionalities may be associated with the input devices / input modalities of the input modalities of the input devices. For example, in Figs. 3a and 3b, examples are shown for an assignment of functionality in a vitreoretinal (VR) and an OCT mode of operation. In the vitreoretinal mode, the functionalities All Lights On/Off, Magnification -, Magnification +, OCT Mode On/Off, Focus - Focus +, VR Synchronized Focus +, VR Synchronized Focus -, X-, X+, Y+, Y- and VR Mode On/Off are assigned to the different modalities of the input device. In the OCT mode, the functionalities OCT Live Mode/Freeze, OCT Optimize, OCT Auto Locate, OCT Mode On/Off, OCT Scan, OCT Next Procedure, OCT Z-, OCT Z+, OCT Left, OCT Right, OCT Up, OCT Down, OCT Save b and OCT Change Joystick State are assigned to the different modalities of the input device. In more formal terms, the input device may have, or may be configured with, two or more sets of functionalities that are associated with the two or more modes of the input device. At the same time, the two or more modes of the input device may be associated with the two or more modes of operation of the microscope system. One (i.e. a single one) of the two or more modes may be active at the input device (at a given time). The system may be configured to generate the visual representation based on the set of functionalities that is associated with the mode that is active at the input device. In other words, the visual representation may be generated depending on which mode is active at the input device / microscope system.

In some cases, the number of different functionalities available surpasses the input modalities provided by the input device or by the one or more input devices. To enable a selection of such functionality, which might not be assigned to any input modality of any of the input devices, the visual overlay may be used. For example, the visual overlay may be used to display, in addition to the visual representation of the control functionality associated with the input device, information on further functionality being available. This may enable the microscope system to provide "unlimited functionality" that is available to the user of the microscope system via the visual overlay. To access the unlimited functionality, the user may request the system to display the available functionality via a further trigger signal. In other words, the system may be configured to obtain a further trigger signal. For example, the further trigger signal may be obtained similar to the trigger signal specified above, and may originate from one of the input devices, from the touch screen, of be generated by the system based on a sensor input. The further trigger signal may indicate a desire of a user of the microscope system to toggle a control functionality associated with the input device. Accordingly, the system may be configured to toggle the control functionality associated with the input device based on the further trigger signal. In other words, the system may be triggered to toggle the control functionality associated with the input device in response to receiving the further trigger signal. For example, the functionality being associated with the respective input modalities of the input device may be changed by toggling the control functionality associated with the input device based on the further trigger signal. Accordingly, the system may be configured to adapt the visual representation (to show the control functionality that is associated with the input device after toggling) after toggling the control functionality associated with the input device.

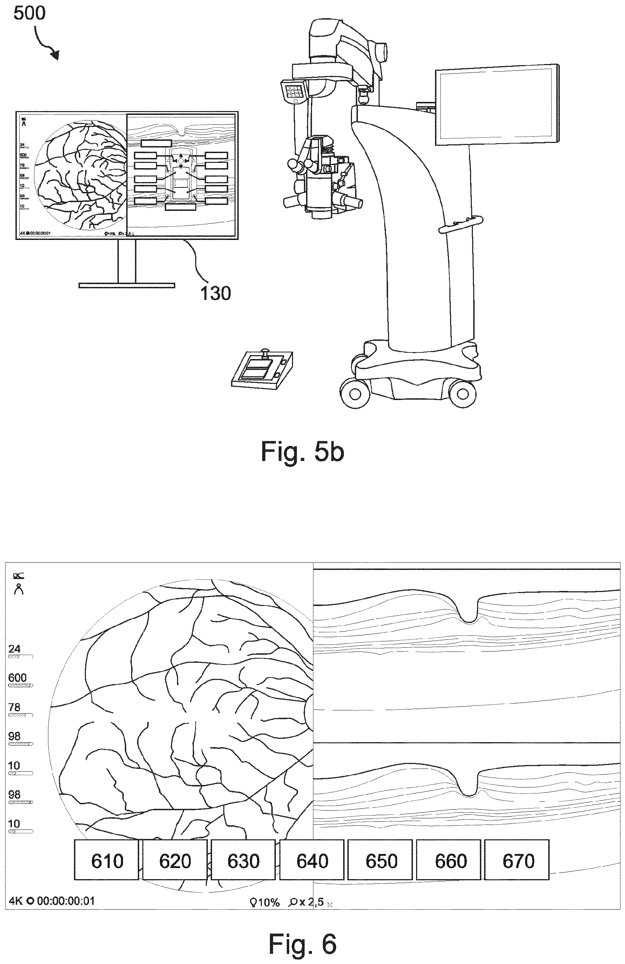

In many cases, the amount of functionality that is available is vast, so that the user/surgeon needs to toggle through a number of functionalities to reach the desired control functionality. Suitably, the control functionality being available may be shown in the visual overlay, so the user/surgeon is made aware of the functionality being available, and can select the desired control functionality assisted by the visual overlay. For example, in Fig. 6, six different control functionalities 610-670 are shown as items on screen, for selection by the user. Accordingly, the system may be configured to generate the visual overlay such, that the visual overlay comprises a visual representation of a control functionality currently associated with the input device (i.e. the visual representation of the control functionality associated with the input device) and a visual representation of a control functionality available for association with the input device after toggling. In other words, the available control functionality, i.e. the control functionality available for association with the input device after toggling, may be displayed as part of the visual overlay. For example, the visual representation of the control functionality available for association with the input device after toggling may be shown side by side with, or instead of, the visual representation of the control functionality currently associated with the input device, e.g. in response to receiving the further trigger signal, or as long as the further trigger signal is received.

In some examples, a smart guidance feature may be provided. For example, the system may be configured to select the control functionality available for association with the input device after toggling based on a progress of a surgery being performed with the help of the surgical microscope system. Furthermore, the system may be configured to generate the visual overlay such, that the surgeon is guided to select control functionality associated with a subsequent step of the surgery being performed.

The system is configured to provide the display signal to a display device 130 of the microscope system (e.g. via the interface 112), the display signal comprising the visual overlay. The display device may be configured to show the visual overlay based on the display signal, e.g. to inject the visual overlay over the view on the sample based on the display signal. For example, the display signal may comprise a video stream or control instructions that comprise the visual overlay, e.g. such that the visual overlay is shown by the respective display device. For example, the display device may be one of an ocular display of the microscope and an auxiliary display of the surgical microscope system. In modern (surgical) microscope systems, the view on the sample is often provided via a display, such as a ocular display, an auxiliary display or a headset display, e.g. using a video stream that is generated based on image sensor data of an optical imaging sensor of the respective microscope. In this case, the visual overlay may be merely overlaid over the video stream. For example, the system may be configured to obtain image sensor data of an optical imaging sensor of the microscope, to generate the video stream based on the image sensor data and to generate the display signal by overlaying the visual overlay over the video stream.

Alternatively, the visual overlay may be overlaid over an optical view of the sample. For example, the ocular eyepieces of the microscope may be configured to provide an optical view on the sample, and the display device may be configured to inject the overlay into the optical view on the sample, e.g. using a one-way mirror or a semi-transparent display that is arranged within an optical path of the microscope. For example, the microscope may be an optical microscope with at least one optical path. One-way mirror(s) may be arranged within the optical path(s), and the visual overlay may be projection onto the one-way mirror(s) and thus overlaid over the view on the sample. In this case, the display device may be a projection device configured to project the visual overlay towards the mirror(s), e.g. such that the visual overlay is reflected towards an eyepiece of the microscope. Alternatively, a display or displays may be used to provide the overlay within the optical path(s) of the microscope. For example, the display device may comprise at least one display being arranged within the optical path(s). For example, the display(s) may be one of a projection-based display and a screen-based display, such as a Liquid Crystal Display (LCD) - or an Organic Light Emitting Diode (OLED)-based display. For example, the display(s) may be arranged within the eyepiece of the optical stereoscopic microscope, e.g. one display in each of the oculars. For example, two displays may be used to turn the oculars of the optical microscope into augmented reality oculars, i.e. an augmented reality eyepiece. Alternatively, other technologies may be used to implement the augmented reality eyepiece/oculars.

The interface 112 may correspond to one or more inputs and/or outputs for receiving and/or transmitting information, which may be in digital (bit) values according to a specified code, within a module, between modules or between modules of different entities. For example, the interface 112 may comprise interface circuitry configured to receive and/or transmit information. In embodiments the one or more processors 114 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. In at least some embodiments, the one or more storage devices 116 may comprise at least one element of the group of a computer readable storage medium, such as an magnetic or optical storage medium, e.g. a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system and microscope system are mentioned in connection with the proposed concept or one or more examples described above or below (e.g. Fig. 2 to 7). The system or microscope system may comprise one or more additional optional features corresponding to one or more aspects of the proposed concept or one or more examples described above or below.

Fig. 2 shows a flow chart of an example of a corresponding method for a microscope system. The method comprises obtaining 210 a trigger signal. The trigger signal indicates a desire of a user of the microscope system to display a visual representation of a control functionality associated with an input device of the microscope system. The method comprises generating 220 based on the trigger signal, a visual overlay comprising the visual representation. The method comprises providing 230 a display signal to a display device of the microscope system, the display signal comprising the visual overlay.

Features described in connection with the system 110 and the microscope system 100 of Figs. 1a and/or 1b may be likewise applied to the method of Fig. 2.

More details and aspects of the method are mentioned in connection with the proposed concept or one or more examples described above or below (e.g. Fig. 1a to 1b, 3 to 7). The method may comprise one or more additional optional features corresponding to one or more aspects of the proposed concept or one or more examples described above or below.

Various aspects of the present disclosure relate to surgical control guidance for microscope interactions.

A foot pedal (also known as footswitch) and handle controls play a crucial role in surgery. While there are some subtle variations among models and manufacturers, the basics of the microscope foot pedal and operation of the microscope are similar. The primary microscope controls are focus, zoom and centration. Additional functions include the ability to turn the microscope light on and off as well as adjust the brightness.

However, during various types of surgery, more than a single foot pedal is being used, so that all of the surgeon's extremities can be busy, for example in basic cataract surgery in ophthalmology, with one foot being placed on the microscope pedal, one foot on a phacoemulsification pedal, one hand interacting with the phacoemulsification handpiece, and the other hand holding the phacoemulsification chopper instrument.

In ophthalmology, typical operation can involve multiple foot pedals - a surgeon may control a foot pedal for the microscope controls, a foot pedal for phacoemulsification to control the phaco irrigation, aspiration and ultrasonic power delivery, a foot pedal for vitrectomy and a foot pedal for laser photocoagulation. In microsurgery, a microscope handle can be programmed with up to 10 functions each to provide a total of 20 functions on both right and left handles.

This brings about several challenges. First, less experienced surgeons may struggle to remember what functions have been programmed on the foot pedal and may sometimes attach, on the microscope, hardcopy printouts or hand drawn illustrations of the controls, for easy referencing during surgery.

With the introduction of advanced surgical guidance technologies to improve patient outcomes and surgeon efficiency, new software applications are integrated into the microscope (e.g. intraocular lens guidance, optical coherence tomography visualization, augmented fluorescence imaging). These surgical guidance applications are activated and controlled by the same foot pedals and handles available on the microscopes. As a result, experienced surgeons may now tackle a new set challenges to re-program additional functions and get re-familiarized with these new controls. A pain point for these surgeons may consist of remembering the associated functions of each foot pedal or handle button for each surgical "mode" (normal, vitreoretinal, OCT functions, etc.), as the programmed functions may differ between modes.

Figs. 3a and 3b show schematic diagrams of functionalities being assigned to a foot pedal of a surgical microscope system. Figs. 3a and 3b illustrate how new functions for advanced surgical guidance applications introduces additional complexity, where experienced surgeons may remember specific functions which have been programmed on the foot pedal and handles, when toggling between surgical modes (e.g. vitreoretinal, OCT functions). The foot pedals 3a and 3b have a plurality of input modalities, comprising a lower left button 301a/b, a lower right button 304a/b, a lower pad comprising a left side 302a/b and a right side 303a/b, a middle left button 305a/b, a middle right button 306a/b, a middle pad comprising a left side 307a/b and a right side 308a/b, a top control stick having a stick that can be moved into a left position 309a/b, top position 310a/b, right position 311a/b and bottom position 312a/b, a top left button 313a/b and a top right button 314a/b. For example, Fig. 3a shows an exemplary assignment of functionalities for a vitreoretinal mode, with the (control) functionality All Lights On/Off being assigned to the lower left button 301a, Magnification - assigned to the left side 302a of the lower pad, Magnification + being assigned to the right side 303a of the lower pad, OCT Mode On/Off being assigned to the lower right button 304a, Focus - being assigned to the middle left button 305a, Focus + being assigned to the middle right button 306a, VR Synchronized Focus + being assigned to the left side 307a of the middle pad, VR Synchronized Focus - being assigned to the right side 308a of the middle pad, X- being assigned to the top control stick being moved to the left position 309a, X+ being assigned to the top control stick being moved to the right position 311a, Y+ being assigned to the top control stick being moved to the top position 310a, Y- being assigned to the top control stick being moved to the bottom position 312a, nothing being assigned to the top left button 313a and VR Mode On/Off being assigned to the top right button 314a.

Fig. 3b shows an exemplary assignment of functionalities for a OCT mode, with the (control) functionality OCT Live Mode/Freeze being assigned to the lower left button 301b, OCT Optimize being assigned to the left side 302b of the lower pad, OCT Auto Locate being assigned to the right side 303b of the lower pad, OCT Mode On/Off being assigned to the lower right button 304b, OCT Scan being assigned to the middle left button 305b, OCT Next Procedure being assigned to the middle right button 306b, OCT Z- being assigned to the left side 307b of the middle pad, OCT Z+ being assigned to the right side 308b of the middle pad, OCT Left being assigned to the top control stick being moved to the left position 309b, OCT Right being assigned to the top control stick being moved to the right position 311b, OCT Up being assigned to the top control stick being moved to the top position 310b, OCT Down being assigned to the top control stick being moved to the bottom position 312b, OCT Save being assigned to the top left button 313b and OCT Change Joystick State being assigned to the top right button 314b.

Limited interactions may be available on the foot pedal and handles, which may limit the number of functions which can be programmed on the controls. For example, in ophthalmology, 4 functionalities may be assigned to various modalities on the handles with the addition of 12-14 functionalities on the foot pedals and in microsurgery, up to a maximum of 10 on the handles. Surgeons may prioritize and program before surgery which functions are needed, with the remaining unselected functions inaccessible.

This may lead to an adoption barrier of new technologies, which require additional functions to be programmed on the foot pedal or handles. Due to the limited interactions available, surgeons often program a dedicated button to switch between "modes" (normal, vitreoretinal, OCT functions, etc.) which leads to complexities as described previously.

Figs. 4a to 4c show schematic diagrams of input devices for a microscope system, and of the input modalities of the input devices. Fig. 4a and 4b show the limited interactions are available on the foot pedal and handles, in ophthalmology, 4 on the handles (clockwise rotation 421 on the left side, counterclockwise rotation 422 on the left side, clockwise rotation 423 on the right side and counterclockwise rotation 424 on the right side) with the addition of 12-14 on the foot pedals (a lower left button 401, a lower right button 404, a lower pad comprising a left side 402 and a right side 403, a middle left button 405, a middle right button 406, a middle pad comprising a left side 407 and a right side 408, a top control stick having a stick that can be moved into a left position 409, top position 410, right position 411 and bottom position 412, a top left button 413 and a top right button 414). Fig. 4c shows the limited interactions that are available on the handles, in microsurgery, up to a maximum of 10 on the handles (a top control stick being moved into a top 431, left 432, right 433 and bottom 434 position, an upper left button 435, an upper right button 436, a lower left button 437, a lower right button 438, a right side button 439 and a back button 440).

Various aspects of the present disclosure provide a surgical guidance overlay of the foot pedal or handles functions on screen (of the display device), either projected on the microscope stand monitor, heads-up display or external operating room monitors. This overlay may display the functions pre-programmed in the handles or footswitch as a reminder to the surgeons during operation. The overlay can be activated by triggering a dedicated button on the handles or footswitch, or via the user interface on the microscope touch screens, or via activation of any of the handles or footswitch functions. In other words, the trigger signal may be generated in response to triggering a dedicated button on the handles or footswitch, or via the user interface on the microscope touch screens, or via activation of any of the handles or footswitch functions. This overlay can be faded in and faded out automatically without interruption to the surgical workflow.

In the following, an overview is given of the proposed concept. In the proposed concept, a surgical guidance overlay, i.e. the visual overlay, of the surgeon's controls, i.e. the visual representation of the control functionality, (via footswitch or handles) is projected on the microscope stand monitor, heads-up 3D display, external operating room monitors or digital viewers. This overlay provides a quick guide for surgeons to reference from during surgery. The overlay can be displayed on the heads-up 3D display or any digital viewers to allow the surgeon to have an undisrupted view from the surgical workflow.

In some cases, the input devices, and the microscope system, may support multiple modes of operation, or short: modes. In this case, the overlay may display the associated functions of the foot pedal buttons and handle interactions according to the surgical mode in use during the operation. The functions displayed may vary across modes.

In some examples, a smart guidance feature may be provided. For example, smart guidance features may be integrated to prompt and guide the surgeon to select the next function in the surgical step, to improve clinical workflow efficiency.

The overlay can also be programmed to display functionalities and guidance from 3rd party accessories, such as pedal controls for the phacoemulsification or vitreoretinal instruments, to allow for a surgical cockpit experience for the surgeon wherein all useful data and controls are displayed to be captured by the surgeon at a single glace.

Regarding a display format being used for the overlay, the overlay may be displayed as a transparent fade in layer upon activation, which may fade out automatically without an additional step needed.

The surgical guidance overlay may be activated by means of triggering a dedicated pre-programmed interaction on the handles or footswitch, or via the touch interfaces on the microscope monitors, or automatically activated when a standard function on the handles or footswitch is triggered. The surgical guidance overlay can also be activated by other means, such as voice recognition, head movement, eye tracking or a touch enabled interface on the foot pedal or handles.

Additionally, the overlay can provide an unlimited selection of functions to the surgeon by displaying on-screen, a list of options which can be rotated through for selection. In other words, the surgical guidance overlay can provide an unlimited range of functionalities which can be displayed on-screen for selection and activation by the surgeon. This allows the surgeons to go beyond what can be pre-programmed on the foot pedal (up to 14 functions) or on the handles (ophthalmology up to 4, microsurgery up to 10 functions).

The additional functions, i.e. the "unlimited functionality" can be displayed as a rotary menu or a pop-up menu on screen to provide a range of functions which can be selected for activation by the foot pedal, handles or touch interfaces connected to the microscope. The additional functions displayed can either be a range of recommended pre-sets based on the surgical mode in operation, or the surgeons can pre-select their favorite functions to be displayed on-screen for activation or deactivation. This is especially beneficial given that more and more functionalities are integrated in microscope systems to guide the surgical workflow, such as optical coherence tomography and intraocular lens guidance. As the surgical trend of tomorrow is increasing moving towards a digital workflow, new software features, such as tool tracking, feature recognition, voice recognition, etc., can be integrated as part of the microscope functionalities, and accessed via the same input devices. These multitude of new functionalities may benefit from the proposed concept to be displayed, selected and activated during surgery. The surgical guidance overlay can provide a platform to enable this, allowing surgeons to operate beyond the limited interactions on handles and foot switches.

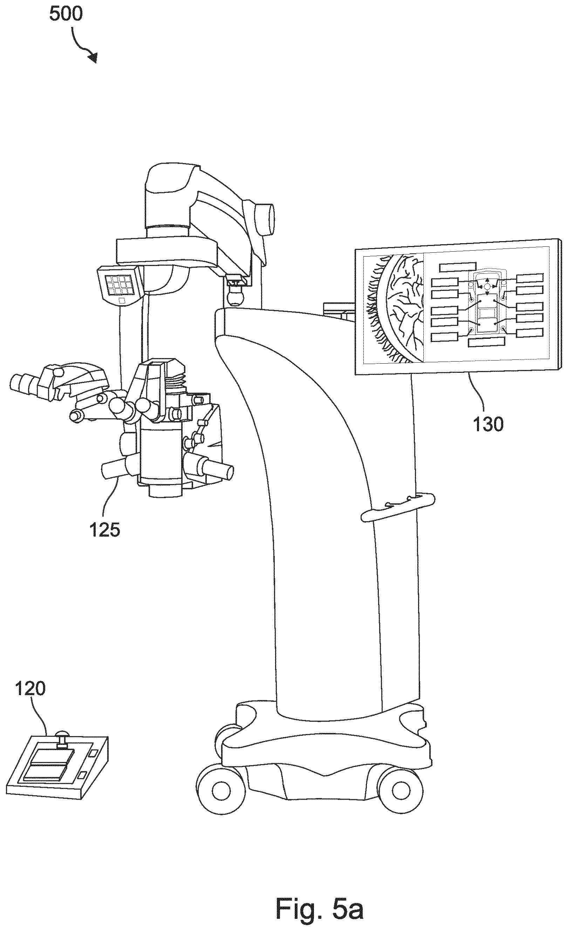

Figs. 5a and 5b show a schematic diagram of a surgical guidance overlay being displayed on a display device of a microscope system. Fig. 5a shows foot pedal 120 or handles functions 125 displayed as an overlay on the microscope display 130 of a microscope system 500. This overlay can be activated by triggering a dedicated button on the handles or footswitch, or via the user interface on the microscope touch screens, or via activation of any of the handles or footswitch functions. Fig. 5b shows foot pedal or handles functions displayed as an overlay on a 3D heads-up surgical display 130 as part of the surgical cockpit experience in another implementation of the microscope system 500, Again, this overlay can be activated by triggering a dedicated button on the handles or footswitch, or via the user interface on the microscope touch screens, or via activation of any of the handles or footswitch functions

As shown in Fig. 6, surgeons are not limited by the number of physical interactions available on the foot pedal and handles, an on-screen options menu provides a virtually unlimited number of functions which can be displayed and selected during surgery. Fig. 6 shows an example of an on-screen menu, with menu items 610 (Activate Tool Tracking), 620 (Activate Digital Filters), 630 (Activate Voice Command), 640 (OCT Playback), 650 OCT Scan, 660 (Photo Capture) and 670 (Video Record).

More details and aspects of the surgical guidance overlay are mentioned in connection with the proposed concept or one or more examples described above or below (e.g. Fig. 1a to 2). The surgical guidance overlay may comprise one or more additional optional features corresponding to one or more aspects of the proposed concept or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

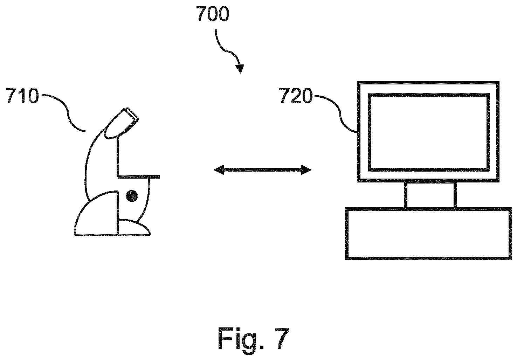

Some embodiments relate to a microscope comprising a system as described in connection with one or more of the Figs. 1 to 6. Alternatively, a microscope may be part of or connected to a system as described in connection with one or more of the Figs. 1 to 6. Fig. 7 shows a schematic illustration of a system 700 configured to perform a method described herein. The system 700 comprises a microscope 710 and a computer system 720. The microscope 710 is configured to take images and is connected to the computer system 720. The computer system 720 is configured to execute at least a part of a method described herein. The computer system 720 may be configured to execute a machine learning algorithm. The computer system 720 and microscope 710 may be separate entities but can also be integrated together in one common housing. The computer system 720 may be part of a central processing system of the microscope 710 and/or the computer system 720 may be part of a subcomponent of the microscope 710, such as a sensor, an actor, a camera or an illumination unit, etc. of the microscope 710.

The computer system 720 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 720 may comprise any circuit or combination of circuits. In one embodiment, the computer system 720 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 720 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 720 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 720 may also include a display device, one or more speakers, and a keyboard and/or controller, which can include a mouse, trackball, touch screen, voice-recognition device, or any other device that permits a system user to input information into and receive information from the computer system 720.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of reference Signs

- 100: Microscope system
- 105: Base unit
- 110: System
- 112: Interface
- 114: One or more processors
- 116: One or more storage devices
- 120: Foot pedal
- 125: Handles
- 130: Display
- 140: Microphone
- 150: Touchscreen
- 160: Microscope
- 170: Arm
- 210: Obtaining a trigger signal
- 220: Generating a visual overlay
- 230: Providing a display signal
- 301a/b: Lower left button
- 302a/b: Left side of a lower pad
- 303a/b: Right side of a lower pad
- 304a/b: Lower right button
- 305a/b: Middle left button
- 306a/b: Middle right button
- 307a/b: Left side of a middle pad
- 308a/b: Right side of a middle pad
- 309a/b: Left position of a control stick
- 310a/b: Top position of a control stick
- 311a/b: Right position of a control stick
- 312a/b: Bottom position of a control stick
- 313a/b: Top left button
- 314a/b: Bottom left button
- 401: Lower left button
- 402: Left side of a lower pad
- 403: Right side of a lower pad
- 404: Lower right button
- 405: Middle left button
- 406: Middle right button
- 407: Left side of a middle pad
- 408: Right side of a middle pad
- 409: Left position of a control stick
- 410: Top position of a control stick
- 411: Right position of a control stick
- 412: Bottom position of a control stick
- 413: Top left button
- 414: Bottom left button
- 421: Clockwise rotation of left handle
- 422: Counterclockwise rotation of left handle
- 423: Clockwise rotation of right handle
- 424: Counterclockwise rotation of right handle
- 431: Top position of top control stick
- 432: Left position of top control stick
- 433: Right position of top control stick
- 434: Bottom position of top control stick
- 435: Upper left button
- 436: Upper right button
- 437: Lower left button
- 438: Lower right button
- 439: Right side button
- 440: Back button
- 500: Microscope system
- 610-670: Menu items
- 700: System
- 710: Microscope
- 720: Computer system

## Claims

1. A system (110; 720) for a microscope system (100; 500; 700), the system (110; 720) comprising one or more processors (114) and one or more storage devices (116), wherein the system is configured to:
obtain a trigger signal, the trigger signal indicating a desire of a user of the microscope system to display a visual representation of a control functionality associated with an input device (120; 125) of the microscope system;
generate, based on the trigger signal, a visual overlay comprising the visual representation; and
provide a display signal to a display device (130) of the microscope system, the display signal comprising the visual overlay.

2. The system according to claim 1, wherein the system is configured to obtain the trigger signal from one of one or more input devices (120; 125) of the microscope system, and to generate the visual overlay with a visual representation of the control functionality associated with the input device the trigger signal is received from.

3. The system according to one of the claims 1 or 2, wherein the system is configured to obtain the trigger signal from a button of the input device.

4. The system according to claim 1, wherein the system is configured to process audio captured via a microphone (140) of the microscope system, and to generate the trigger signal based on one or more keywords spoken by the user within the captured audio,
or wherein the system is configured to obtain the trigger signal via a touch-screen (150) of the microscope system.

5. The system according to one of the claims 1 to 4, wherein the visual representation represents a control functionality associated with a foot pedal (120), with a handle (125) of the microscope system, with a mouth switch of the microscope system, or with an eye-tracking system of the microscope system.

6. The system according to one of the claims 1 to 5, wherein the input device has two or more sets of functionalities that are associated with two or more modes of the input device, with one of the two or more modes being active at the input device, wherein the system is configured to generate the visual representation based on the set of functionalities that is associated with the mode that is active at the input device.

7. The system according to one of the claims 1 to 6, wherein the visual representation of the control functionality comprises a visual representation of the input device the control functionality is associated with.

8. The system according to one of the claims 1 to 7, wherein the system is configured to obtain a further trigger signal, the further trigger signal indicating a desire of a user of the microscope system to toggle a control functionality associated with the input device, wherein the system is configured to toggle the control functionality associated with the input device based on the further trigger signal, and wherein the system is configured to adapt the visual representation after toggling the control functionality associated with the input device.

9. The system according to claim 8, wherein the system is configured to generate the visual overlay such, that the visual overlay comprises a visual representation of a control functionality currently associated with the input device and a visual representation of a control functionality available for association with the input device after toggling.

10. The system according to one of the claims 1 to 9, wherein the visual representation represents a plurality of control functionalities associated with a plurality of input modalities of the input device.

11. A microscope system 100 comprising the system (110) according to one of the claims 1 to 10, an input device (120; 125), and a display device (130).

12. The microscope system according to claim 11, wherein the input device is one of a foot pedal (120), a handle (125), a mouth switch or an eye-tracking system of the microscope system.

13. The microscope system according to one of the claims 11 or 12, wherein the input device comprises a plurality of input modalities, the plurality of input modalities comprising at least one of one or more buttons, one or more switches, one or more rotary controls, and one or more control sticks.

14. A method for a microscope system, the method comprising:
obtaining (210) a trigger signal, the trigger signal indicating a desire of a user of the microscope system to display a visual representation of a control functionality associated with an input device of the microscope system;
generating (220) based on the trigger signal, a visual overlay comprising the visual representation; and
providing (230) a display signal to a display device of the microscope system, the display signal comprising the visual overlay.

15. A computer program with a program code for performing the method according to claim 14 when the computer program is executed on a processor.
